(19) Europäisches Patentamt
European Patent Office
Office européen des brevets

(11) Publication number: 0 223 238 B1

# (12) EUROPEAN PATENT SPECIFICATION

(45) Date of publication of patent specification: 14.08.91 (51) Int. Cl.[5]: C08G 65/32

(21) Application number: 86116015.8

(22) Date of filing: 18.11.86

The file contains technical information submitted after the application was filed and not included in this specification

## (54) Process for preparing perfluoropolyethers of regulated molecular weight having neutral and functional end groups.

(30) Priority: 19.11.85 IT 2290285
20.11.85 IT 2292285

(43) Date of publication of application:
27.05.87 Bulletin 87/22

(45) Publication of the grant of the patent:
14.08.91 Bulletin 91/33

(84) Designated Contracting States:
BE DE ES FR GB IT NL SE

(56) References cited:
EP-A- 0 167 258
BE-A- 764 110
DE-A- 1 816 752
GB-A- 1 192 238
US-A- 4 523 039

(73) Proprietor: AUSIMONT S.p.A.
31, Foro Buonaparte
I-20121 Milano(IT)

(72) Inventor: Caporiccio, Gerardo
13, Via E. Filiberto
I-20149 Milan(IT)
Inventor: Viola, Gian Tommaso
13, Via Pignocchi
I-48015 Cervia Ravenna(IT)
Inventor: Marchionni, Giuseppe
8, Via Vallisneri
I-20133 Milan(IT)
Inventor: De Iorio, Pio
6, Via delle Ande
I-20151 Milan(IT)
Inventor: Tasca, Adriana
9, Via Bognetti
I-20141 Milan(IT)

(74) Representative: Barz, Peter, Dr. et al
Patentanwälte Dipl.-Ing. G. Dannenberg Dr. P. Weinhold, Dr. D. Gudel Dipl.-Ing. S. Schubert, Dr. P. Barz Siegfriedstrasse 8
W-8000 München 40(DE)

EP 0 223 238 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to the preparation of low molecular weight perfluoropolyethers by a process for the cracking of high molecular weight perfluoropolyethers obtained from the photochemical oxidation of perfluoroolefins or by anionic polymerization of their epoxides.

The preparation of perfluoropolyethers, which are stable at ambient temperature and are obtained from perfluoropolyperoxides, is, for example, described in GB-A-1 226 566 and 1 104 482.

By using perfluoropropene as a starting material it is possible to obtain compounds which, after thermal or photochemical reduction of the peroxy precursor, have the general formula:

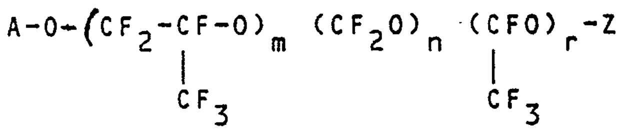
(I)

wherein A is a neutral end group of the type
$-CF_3$, $-C_2F_5$ or $-C_3F_7$
and Z is an acid group

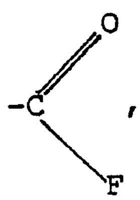 , or 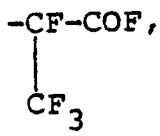 , or $-CF_2COF$

or a group $-CO-CF_3$. The monomeric units with the indices m, n and r are statistically distributed a long the chain. The values of m, n and r vary from 0 to 200 (their sum always being greater than zero) and depend on the photooxidation reaction parameters.

By neutralization of the compounds of formula (I) by treatment with gaseous fluorine at temperatures ranging from 100 to 200 °C, the corresponding neutral products are obtained, wherein Z is converted to a group A' having the same meaning as A.

By using tetrafluoroethylene as a starting material, perfluoropolyethers are obtained which, after thermal or photochemical reduction of the peroxy precursor, have the general formula:

$$Z'-O-(CF_2CF_2O)_p\ (CF_2O)_q-Z \qquad (II)$$

wherein Z and Z' are acid groups 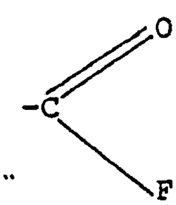 or $-CF_2COF$

or $-CF_3$ or $-C_2F_5$. The monomeric units with the indices p and q are statistically distributed a long the chain; the values thereof depend on the photooxidation reaction parameters and are such that the p/q ratio ranges from 0.5 to 2.

By neutralization with fluorine - as already indicated for the compounds of formula (I) - corresponding neutral products are obtained in which Z' and Z, which are the same or different, are $-CF_3$ or $-C_2F_5$.

Finally, perfluoropolyethers prepared by polymerization in the presence of anionic catalysts of perfluoropropene epoxide are also known, as is, for example, described in US-A-3 250 808. The general formula of these products is:

$$CF_3CF_2CF_2O-(CF(CF_3)-CF_2O)_s-Y \qquad (III)$$

wherein Y is an acyl fluoride group 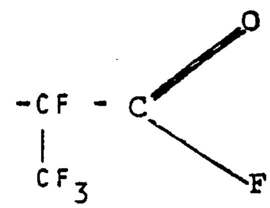

and s is an integer greater than zero. (The above formula I also covers compounds of formula III.)

By neutralization with fluorine, neutral compounds wherein Y is $-C_2F_5$ are obtained.

The processes for the preparation of such perfluoropolyethers lead to a distribution of the molecular weight of the products with a tendency towards high molecular weight and it is difficult to find practical applications for such products.

GB-A-1,192,238 discloses a process for the modification of certain perfluoropolyethers, i.e., the (fluoroformate) end groups thereof, in the optional presence of, e.g., compounds of Zn, Cd, Al or Fe. Said compounds are preferably employed in stoichiometric amounts.

EP-A-167 258, a document relevant under Art. 54(3) EPC for all designated states except IT and ES discloses a process for the cracking of high molecular weight perfluoropolyethers by cracking in the presence of 0.1 to 2% by weight of Al, Ti, V, Co and Ni fluorides or oxyfluorides, at temperatures ranging from 150 to 380 °C.

Said treatment results in perfluoropolyethers of lower and controlled molecular weight, which are the products that can be used best in practice. It is well-known that the low molecular weight products are used as operative and testing fluids in electronics. The medium molecular weight products are utilized as operative fluids in the vacuum sector.

It has now, surprisingly, been found that it is possible to carry out such a cracking process by using catalysts which differ from the ones indicated in the above-mentioned patent application.

Thus, the present invention provides a process for the cracking of perfluoropolyethers of formulae I, II (and III), carried out at temperatures ranging from 150 to 380 °C in the presence of from 0.1 to 10% by weight, based on the weight of the starting perfluoropolyether, of catalysts comprising:

- Oxides of the following metals: Ti, Cr, Mn, Fe, Co, Ni, V, Cu, Mo, Sn, Sb, Zr, Zn
- Fluorides and oxyfluorides of the following metals:
  Cr, Mn, Fe, Cu, Mo, Sn, Sb, Zr, Zn.

For all designated states except IT and ES there additionally is the proviso that when only oxides of Ti, V, Co and Ni are employed (individually or as combination of two or more thereof), the total amount thereof is such that by their reaction with (liberated) fluorine only less than 0.1% or more than 2% by weight of the corresponding fluorides and/or oxyfluorides are formed.

The fluorides and that oxyfluorides can also be prepared in situ starting from halides (different from the fluorides) and operating in the presence of fluorine.

By varying temperature, time, amount of catalyst and type of catalyst, it is possible to obtain perfluoropolyethers having a molecular weight lower than that of the starting materials and having the desired molecular weight.

As already indicated before, it is preferable to use, as starting perfluoropolyethers, the products of formulae I, II and III having neutral end groups. It is, however, also possible to utilize the perfluoropolyethers of formulae I, II and III containing acid and/or ketonic end groups.

In this case, higher amounts of catalyst are to be used. The catalyst is, preferably, added when the perfluoropolyether has already reached the treatment temperature and is employed for prolonged periods of time.

Mixtures of the cited catalysts are also useful.

In the presence of the above-mentioned catalysts, the cracking of the perfluoropolyether chain occurs independently of the monomeric structural sequences which are present in the chain, with the formation of shorter chains having end groups of the type:

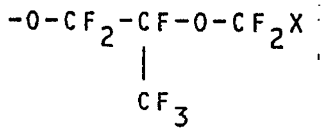

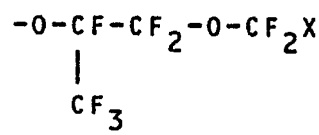

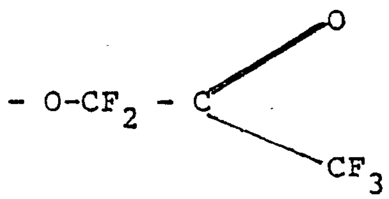

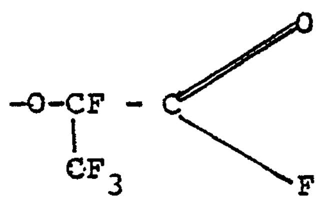

wherein X is -F, $-CF_3$, $C_2F_5$ when the starting perfluoropolyether is derived from $C_3F_6$; when the perfluoropolyether is derived from $C_2F_4$, the end groups obtained are of the type:

$-OCF_3$, $-OCF_2CF_3$, $-O-COF$, $-CF_2COF$.

A particularly advantageous embodiment of the present invention is the combination of the chemical cracking treatment with a fractionation treatment, for example, by means of distillation or flash separation or molecular distillation of the dissociation products. Such treatment is carried out immediately after cracking or simultaneously with it.

In the former case, the operation conditions during the cracking step should be controlled in such a manner that the degree of dissociation is not too high in order to prevent a considerable formation of products having a molecular weight that is too low. The dissociation product, either as a liquid or as a vapour, is then subjected to a fractionation step, for example, by means of distillation or of the methods mentioned above, during which step these products, having the desired mean molecular weight, are separated while the tail fraction, consisting of products still having a molecular weight that is too high, is recycled to the cracking reactor. The process is preferably conducted continuously, thus obtaining a product with a low dispersion index for each desired mean molecular weight.

In the latter case, the temperature and pressure in the reactor should be such that the reaction mixture is kept boiling and the distillate is subjected to distillation in a column in order to obtain, at the top, the products having a molecular weight that is sufficiently low, i.e., not exceeding the predetermined value, while the products with a molecular weight that is too high are continuously recycled to the cracking reactor.

In this way, the light products are removed at once from the reaction mixture, thus preventing their further dissociation which would give rise to products with a molecular weight that is lower than that to be obtained and to low-boiling products and losses.

Both methods are suited to provide, with high yields, fractions having the desired mean molecular weight and a narrow molecular weight distribution while obtaining a high degree of dissociation of the high molecular weight products and, at the same time, avoiding or reducing to a minimum a dissociation of the products which already have the desired molecular weight.

It has been found that the specific process wherein, during the chemical cracking, the products having a molecular weight that is sufficiently low, are continuously separated from the reaction mixture can be applied advantageously also to the chemical cracking treatment described in the above-mentioned EP-A-167258. Some of the following examples (examples 4 to 6) illustrate this particular embodiment by using the cracking catalysts described in EP-A-167258.

The same is, however, true for the catalysts used for cracking in the present invention.

The following examples illustrate the present invention but do not limit it in any way.

EXAMPLE 1

500 g of perfluoropolyether (PFPE), prepared by photooxidation of $C_2F_4$ and, subsequently, subjected

to thermal reduction of the peroxy content and to neutralization with elemental fluorine having a mean molecular weight (MW) of 10250 a.m.u. were introduced into a 1200 ml reactor equipped with a stirrer, a reflux cooler and a $CO_2$ trap and heated electrically. After the addition of 10 g of $TiO_2$ the temperature was brought to 200 °C and the mixture was allowed to react for 30 minutes. At the end of the reaction and after filtration 401 g of PFPE having a molecular weight of 3550 a.m.u. and an acidity of 0.3 meq KOH/g were obtained.

EXAMPLE 2

Using the same equipment as in the preceding example, 20 g of titanium dioxide were added to 500 g of perfluoropolyether obtained by photooxidation of $C_2F_4$ and subsequent reduction and fluorination, as described in example 1, having an MW of 15000 a.m.u.

The reaction was conducted at 220 °C for 40 minutes by working as in example 1. 202 g of perfluoropolyether having a molecular weight of 3500 a.m.u. were obtained. 200 g of perfluoropolyether with a molecular weight of 1500 a.m.u. were collected in the $CO_2$ trap.

EXAMPLE 3

Into a nickel reactor equipped with a stirrer, heating elements and a bubbler, 1000 g of neutral and non-peroxy PFPE, obtained from $C_2F_4$, having a molecular weight of 10250 a.m.u. were introduced, along with 10 g of a mixture of $FeCl_2$, $CoCl_2$ and $CrCl_3$ in a ratio of 3:1:1.

After flushing the reactor with $N_2$ (10 l/h) the temperature was brought to 180 °C, whereafter $N_2$ was replaced by gaseous fluorine (10 l/h). After a five hour-reaction 900 g of a partially acid PFPE (0.02 meq KOH/g), having a molecular weight of 8500 a.m.u., were obtained. The product thus obtained was subjected, after filtration, to neutralization by treating it with a fluorine stream (10 l/h) for 5 hours at 200 °C in a glass reactor. The resulting product was completely neutral and had a mean molecular weight of 8500.

EXAMPLE 4 (reference example)

A perfluoropolyether (type Fomblin Y® of Montedison) stream of 0.5 kg/h, having a kinematic viscosity of 325 $mm^2/s$ (cSt) at 20 °C was fed to a reactor having a reaction volume of 4 liter and an $AlF_3$ concentration of 0.3% by weight. The temperature in the reactor was maintained at about 280 °C. The pressure was kept at atmospheric pressure. The feed for the separation section, consisting of a flash chamber, was drawn from the reactor as a liquid. Pressure and temperature in the flash chamber were maintained at 1 mbar and 285 °C respectively.

The distilled and condensed stream had a viscosity of 93 $mm^2/s$ (cSt). The yield of the distilled fraction was 88%. The residue was continuously recycled to the reactor (about 10 kg/h with a ratio of feed stream to stream recycled to the reactor of about 20). A non-condensable gas and low-boiling products were obtained as distillation by-products.

The distilled product was then subjected to a batchwise distillation.

The fractions indicated in table 1 were obtained. The standard deviation of the distribution of natural logarithms of the viscosity was 1.19.

EXAMPLE 5 (reference example)

A discontinuous test was carried out in a reaction volume of 0.9 liters, having a mean $AlF_3$ concentration of 0.3% by weight. The product charged to the reactor was the same product as that used in example 4. The reactor was kept at atmospheric pressure and a temperature of 280 °C.

The test was carried out for a time sufficient to obtain a product having a viscosity of 93 $mm^2/s$ (cSt).

The yield was 83%, the remaining portion being composed of non-condensable and low-boiling gases.

The resulting product was then subjected to a batchwise distillation using the same apparatus and mode of operation as in example 4.

Six fractions were obtained.

Yields and viscosities of the fractions obtained are reported in table 1.

The standard deviation of the distribution of the natural logarithms of viscosities was 1.65.

A comparison between example 4 and example 5 shows that the molecular weight distribution is narrower when the process is carried out continuously and the products having a high molecular weight are recycled.

EXAMPLE 6 (reference example)

1000 g of perfluoropolyether (Fomblin Y®, produced by Montedison), having a mean molecular weight of about 4500 were placed into a glass reactor of 2 liter volume, equipped with a glass distillation column (number of theoretical plates: 8). $AlF_3$ was added to the perfluoropolyether feed in an amount of 0.5% by weight, based on the weight of the perfluoropolyether.

The entire mixture was heated to a reaction temperature of 300 °C and the above-mentioned perfluoropolyether was then continuously fed to the reactor at a flow rate of 510 g/h.

The mixture of volatile products generated by the reacting mass was passed directly to the fractionation column where a reflux ratio of 3:1 was maintained.

The temperature at the top of the column was maintained at 200 °C. A distillate having an average molecular weight of 1100 and a dispersion index of 1.51, in an amount corresponding to 75%, of the charged product was thus obtained. Low-boiling products and non-condensable gases were obtained as distillation by-products. The process was carried out continuously during 24 hours without varying the operation conditions.

TABLE 1

| Example 4 | | Example 5 | |
|---|---|---|---|
| $mm^2/s$ (cSt) | % by wt. ($\omega_i$) | $mm^2/s$ (cSt) | % by wt. ($\omega_i$) |
| 10.4 | 17 | 4.6 | 15.2 |
| 49.4 | 15 | 32.5 | 17.83 |
| 85.3 | 11.5 | 69 | 9.11 |
| 133 | 18.7 | 121 | 16.6 |
| 196 | 12.21 | 234 | 16.1 |
| 329 | 25.7 | 655.9 | 25 |
| $\sigma = 1.187$ | | $\sigma = 1.65$ | |

$$\left( \frac{\sum_{1}^{n} {}_i \, (\ln \eta_i)^2 \, \omega_i - (\ln \bar{\eta})^2}{100} \right)^{1/2} ;$$

$\omega_i$ = % by weight of the distilled fraction

$\eta_i$ = kinematic viscosity at 20°C in $mm^2/s$ (cSt) of fraction i

$n_i$ = distilled fractions (i varies from 1 to 6)

$$\ln \bar{\eta} = \sum_{1}^{n} {}_i \, \omega_i \, \ln \eta_i$$

## Claims

### Claims for the following Contracting States : DE, GB, FR, NL, SE, BE.

1. A process for the cracking of perfluoropolyethers obtained from the photooxidation of perfluoroolefins or by anionic polymerization of their epoxides, and selected from the following classes of per-

fluoropolyethers:

$A-O-(CF_2-CF(CF_3)-O)_m(CF_2O)_n(CF(CF_3)O)_r-Z$ (I)

wherein A is a perfluoroalkyl end group of the type $CF_3$, $C_2F_5$ or $C_3F_7$ and Z is an acid group selected from:

$-COF$, $-CF(CF_3)COF$ and $-CF_2COF$

or a group $-CO-CF_3$, or is A, the monomeric units with the indices m, n and r being statistically distributed along the chain and m, n and r varying from 0 to 200, their sum being always greater than 0;

$Z'-O-(CF_2CF_2O)_p(CF_2O)_q-Z$ (II)

wherein Z and Z' represent acid groups $-COF$ or $-CF_2COF$, or are $CF_3$ or $C_2F_5$, the monomeric units with the indices p and q being statistically distributed along the chain and p and q being such that the ratio p/q ranges from 0.5 to 2;

comprising heating said perfluoropolyethers to a temperature from 150 to 380 °C in the presence of 0.1 to 10% by weight of catalysts, comprising
oxides of Ti, Cr, Mn, Fe, Co, Ni, V, Cu, Mo, Sn, Sb, Zr or Zn; or
fluorides or oxyfluorides of Cr, Mn, Fe, Cu, Mo, Sn, Sb, Zr or Zn;
provided that when only oxides of Ti, V, Co and Ni are employed (individually or as combination of two or more thereof), the total amount thereof is such that by their reaction with fluorine only less than 0.1% or more than 2% by weight of the corresponding fluorides and/or oxyfluorides are formed.

2. The process according to claim 1, wherein the fluoride or oxyfluoride is prepared in situ by reaction of the corresponding halide, other than fluoride, in the presence of fluorine.

3. The process according to any one of claims 1 and 2, wherein the temperature and pressure in the cracking reactor are adjusted so as to maintain the reaction mixture boiling and the distillate is subjected to fractionation in a column in order to obtain at the top a distillate having the predetermined molecular weight, while the fraction having a higher molecular weight is recycled to the cracking reactor.

4. The process according to claim 3, wherein the cracking catalyst is selected from fluorides or oxyfluorides of the metals indicated in claim 1.

**Claims for the following Contracting States : IT, ES.**

1. A process for the cracking of perfluoropolyethers obtained from the photooxidation of perfluoroolefins or by anionic polymerization of their epoxides, and selected from the following classes of perfluoropolyethers:

$A-O-(CF_2-CF(CF_3)-O)_m(CF_2O)_n(CF(CF_3)O)_r-Z$ (I)

wherein A is a perfluoroalkyl end group of the type $CF_3$, $C_2F_5$ or $C_3F_7$ and Z is an acid group selected from:

$-COF$, $-CF(CF_3)COF$ and $-CF_2COF$

or a group $-CO-CF_3$, or is A, the monomeric units with the indices m, n and r being statistically distributed along the chain and m, n and r varying from 0 to 200, their sum being always greater than 0;

$Z'-O-(CF_2CF_2O)_p(CF_2O)_q-Z$ (II)

wherein Z and Z' represent acid groups -COF or $-CF_2COF$, or are $CF_3$ or $C_2F_5$, the monomeric units with the indices p and q being statistically distributed along the chain and p and q being such that the ratio p/q ranges from 0.5 to 2;

comprising heating said perfluoropolyethers to a temperature from 150 to 380 °C in the presence of 0.1 to 10% by weight of catalysts, comprising
oxides of Ti, Cr, Mn, Fe, Co, Ni, V, Cu, Mo, Sn, Sb, Zr or Zn; or
fluorides or oxyfluorides of Cr, Mn, Fe, Cu, Mo, Sn, Sb, Zr or Zn.

2. The process according to claim 1, wherein the fluoride or oxyfluoride is prepared in situ by reaction of the corresponding halide, other than fluoride, in the presence of fluorine.

3. The process according to any one of claims 1 and 2, wherein the temperature and pressure in the cracking reactor are adjusted so as to maintain the reaction mixture boiling and the distillate is subjected to fractionation in a column in order to obtain at the top a distillate having the predetermined molecular weight, while the fraction having a higher molecular weight is recycled to the cracking reactor.

4. The process according to claim 3, wherein the cracking catalyst is selected from fluorides or oxyfluorides of the metals indicated in claim 1.

**Revendications**
**Revendications pour les Etats contractants suivants : BE, DE, FR, GB, NL, SE.**

1. Un procédé de craquage de perfluoropolyéthers obtenus par photo-oxydation de perfluoro-oléfines ou par polymérisation anionique de leurs époxydes, et choisis parmi les classes suivantes de perfluoropolyéthers:

$A-O-[CF_2-CF(CH_3)O]_m(CF_2O)_n[CF(CF_3)O]_r-Z$ (I)

dans laquelle:
A est un groupe terminal perfluoroalkyle du type $CF_3$, $C_2F_5$ ou $C_3F_7$; et
Z est un groupe acide choisi parmi:
$-COF$, $-CF(CF_3)COF$ et $-CF_2COF$
ou un groupe $-CO-CF_3$, ou est A;
les monomères avec les indices m, n et r étant distribués statistiquement le long de la chaîne et m, n et r variant de 0 à 200, leur somme étant toujours supérieure à 0;

$Z'-O-(CF_2CF_2O)_p(CF_2O)_q-Z$ (II)

dans laquelle:
Z et Z' représentent des groupes acides -COF ou $-CF_2COF$, ou sont $CF_3$ ou $C_2F_5$;
les monomères avec les indices p et q étant distribués statistiquement le long de la chaîne et p et q étant tels que le rapport p/q varie de 0,5 à 2;

comprenant le chauffage desdits perfluoropolyéthers à une température de 150 à 380 °C en présence de 0,1 à 10% en poids de catalyseurs comprenant:
. des oxydes de Ti, Cr, Mn, Fe, Co, Ni, V, Cu, Mo, Sn, Sb, Zr ou Zn; ou
. des fluorures ou oxyfluorures de Cr, Mn, Fe, Cu, Mo, Sn, Sb, Zr ou Zn;

pourvu que, lorsque seuls les oxydes de Ti, V, Co et Ni sont utilisés (individuellement ou en combinaison de deux ou plusieurs d'entre eux), la quantité totale de ceux-ci est telle que, par leur réaction avec du fluor, les fluorures et/ou oxyfluorures correspondants sont formés à raison de seulement moins de 0,1% ou plus de 2% en poids.

2. Le procédé selon la revendication 1, dans lequel le fluorure ou l'oxyfluorure est préparé in situ par réaction de l'halogénure correspondant, autre que le fluorure, en présence de fluor.

3. Le procédé selon l'une quelconque des revendications 1 et 2, dans lequel la température et la pression dans le réacteur de craquage sont ajustées de façon à maintenir le mélange réactionnel au point d'ébullition et le distillat est soumis à un fractionnement dans un colonne afin d'obtenir en tête un

distillat ayant le poids moléculaire prédéterminé, tandis que la fraction ayant un poids moléculaire plus élevé est recyclée vers le réacteur de craquage.

**4.** Le procédé selon la revendication 3, dans lequel le catalyseur de craquage est choisi parmi les fluorures ou oxyfluorures des métaux indiqués dans la revendication 1.

**Revendications pour les Etats contractants suivants : IT, ES.**

**1.** Un procédé de craquage de perfluoropolyéthers obtenus par photo-oxydation de perfluoro-oléfines ou par polymérisation anionique de leurs époxydes, et choisis parmi les classes suivantes de perfluoropolyéthers:

$A\text{-}O\text{-}[CF_2\text{-}CF(CH_3)O]_m(CF_2O)_n[CF(CF_3)O]_r\text{-}Z$ (I)

dans laquelle:

A est un groupe terminal perfluoroalkyle du type $CF_3$, $C_2F_5$ ou $C_3F_7$; et

Z est un groupe acide choisi parmi:

$-COF$, $-CF(CF_3)COF$ et $-CF_2COF$

ou un groupe $-CO\text{-}CF_3$, ou est A;

les monomères avec les indices m, n et r étant distribués statistiquement le long de la chaîne et m, n et r variant de 0 à 200, leur somme étant toujours supérieure à 0;

$Z'\text{-}O\text{-}(CF_2CF_2O)_p(CF_2O)_q\text{-}Z$ (II)

dans laquelle:

Z et Z' représentent des groupes acides $-COF$ ou $-CF_2COF$, ou sont $CF_3$ ou $C_2F_5$;

les monomères avec les indices p et q étant distribués statistiquement le long de la chaîne et p et q étant tels que le rapport p/q varie de 0,5 à 2;

comprenant le chauffage desdits perfluoropolyéthers à une température de 150 à 380°C en présence de 0,1 à 10% en poids de catalyseurs comprenant:

. des oxydes de Ti, Cr, Mn, Fe, Co, Ni, V, Cu, Mo, Sn, Sb, Zr ou Zn; ou

. des fluorures ou oxyfluorures de Cr, Mn, Fe, Cu, Mo, Sn, Sb, Zr ou Zn.

**2.** Le procédé selon la revendication 1, dans lequel le fluorure ou l'oxyfluorure est préparé in situ par réaction de l'halogénure correspondant, autre que le fluorure, en présence de fluor.

**3.** Le procédé selon l'une quelconque des revendications 1 et 2, dans lequel la température et la pression dans le réacteur de craquage sont ajustées de façon à maintenir le mélange réactionnel au point d'ébullition et le distillat est soumis à un fractionnement dans un colonne afin d'obtenir en tête un distillat ayant le poids moléculaire prédéterminé, tandis que la fraction ayant un poids moléculaire plus élevé est recyclée vers le réacteur de craquage.

**4.** Le procédé selon la revendication 3, dans lequel le catalyseur de craquage est choisi parmi les fluorures ou oxyfluorures des métaux indiqués dans la revendication 1.

**Patentansprüche**

**Patentansprüche für folgende Vertragsstaaten : BE, DE, FR, GB, NL, SE.**

**1.** Verfahren zum Cracken von Perfluorpolyethern, die durch die Photooxidation von Perfluorolefinen oder durch anionische Polymerisation ihrer Epoxide erhalten werden und aus den folgenden Klassen von Perfluorpolyethern ausgewählt sind:

$A\text{-}O\text{-}(CF_2\text{-}CF(CF_3)\text{-}O)_m(CF_2O)_n(CF(CF_3)O)_r\text{-}Z$ (I)

worin A eine Perfluoralkyl-Endgruppe des Typs $CF_3$, $C_2F_5$ oder $C_3F_7$ darstellt und Z eine Säuregruppe ist,
ausgewählt aus:

$-COF$, $-CF(CF_3)COF$ und $-CF_2COF$

oder eine Gruppe $-CO-CF_3$ oder A bedeutet, die monomeren Einheiten mit den Indizes m, n und r statistisch entlang der Kette verteilt sind und m, n und r von 0 bis 200 variieren, wobei die Summe immer größer als 0 ist;

$$Z'-O-(CF_2CF_2O)_p(CF_2O)_q-Z \quad (II)$$

worin Z und Z' Säuregruppen $-COF$ oder $-CF_2cOF$ repräsentieren oder $CF_3$ oder $C_2F_5$ bedeuten, wobei die monomeren Einheiten mit den Indizes p und q statistisch entlang der Kette verteilt sind und p und q solcherart sind, daß das Verhältnis p/q von 0,5 bis 2 reicht;

umfassend das Erhitzen der Perfluorpolyether auf eine Temperatur von 150 bis 380 °C in Gegenwart von 0,1 bis 10 Gewichtsprozent Katalysatoren, umfassend

Oxide von Ti, Cr, Mn, Fe, Co, Ni, V, Cu, Mo, Sn, Sb, Zr oder Zn; oder
Fluoride oder Oxifluoride von Cr, Mn, Fe, Cu, Mo, Sn, Sb, Zr oder Zn;
mit der Maßgabe, daß falls nur Oxide von Ti, V, Co und Ni (einzeln oder als Kombination von zwei oder mehreren davon) eingesetzt werden, deren Gesamtmenge so ist, daß durch ihre Reaktion mit Fluor nur weniger als 0,1 Gew.-% oder mehr als 2 Gew.-% der entsprechenden Fluoride und/oder Oxyfluoride gebildet werden.

2. Verfahren nach Anspruch 1, worin das Fluorid oder Oxifluorid in situ durch die Umsetzung des entsprechenden Halogenids, außer Fluorid, in Gegenwart von Fluor hergestellt wird.

3. Verfahren nach irgendeinem der Ansprüche 1 und 2, worin die Temperatur und der Druck im Crack-Reaktor so eingestellt sind, daß die Reaktionsmischung am Sieden gehalten wird, und das Destillat der Fraktionierung in einer Säule unterworfen wird, um am Kopf ein Destillat mit dem vorbestimmten Molekulargewicht zu erhalten, während die Fraktion mit einem größeren Molekulargewicht in den Crack-Reaktor zurückgeführt wird.

4. Verfahren nach Anspruch 3, worin der Crack-Katalysator aus Fluoriden oder Oxifluoriden der in Anspruch 1 angegebenen Metalle ausgewählt ist.

**Patentansprüche für folgende Vertragsstaaten : IT, ES.**

1. Verfahren zum Cracken von Perfluorpolyethern, die durch die Photooxidation von Perfluorolefinen oder durch anionische Polymerisation ihrer Epoxide erhalten werden und aus den folgenden Klassen von Perfluorpolyethern ausgewählt sind:

$$A-O-(CF_2-CF(CF_3)-O)_m(CF_2O)_n(CF(CF_3)O)_r-Z \quad (I)$$

worin A eine Perfluoralkyl-Endgruppe des Typs $CF_3$, $C_2F_5$ oder $C_3F_7$ darstellt und Z eine Säuregruppe ist,
ausgewählt aus:

$-COF$, $-CF(CF_3)COF$ und $-CF_2COF$

oder eine Gruppe $-CO-CF_3$ oder A bedeutet, die monomeren Einheiten mit den Indizes m, n und r statistisch entlang der Kette verteilt sind und m, n und r von 0 bis 200 variieren, wobei die Summe immer größer als 0 ist;

$$Z'-O-(CF_2CF_2O)_p(CF_2O)_q-Z \quad (II)$$

worin Z und Z' Säuregruppen $-COF$ oder $-CF_2COF$ repräsentieren oder $CF_3$ oder $C_2F_5$ bedeuten, wobei die monomeren Einheiten mit den Indizes p und q statistisch entlang der Kette verteilt sind und p und q solcherart sind, daß das Verhältnis p/q von 0,5 bis 2 reicht;

umfassend das Erhitzen der Perfluorpolyether auf eine Temperatur von 150 bis 380 °C in Gegenwart

von 0,1 bis 10 Gewichtsprozent Katalysatoren, umfassend

Oxide von Ti, Cr, Mn, Fe, Co, Ni, V, Cu, Mo, Sn, Sb, Zr oder Zn; oder
Fluoride oder Oxifluoride von Cr, Mn, Fe, Cu, Mo, Sn, Sb, Zr oder Zn.

**2.** Verfahren nach Anspruch 1, worin das Fluorid oder Oxifluorid in situ durch die Umsetzung des entsprechenden Halogenids, außer Fluorid, in Gegenwart von Fluor hergestellt wird.

**3.** Verfahren nach irgendeinem der Ansprüche 1 und 2, worin die Temperatur und der Druck im Crack-Reaktor so eingestellt sind, daß die Reaktionsmischung am Sieden gehalten wird, und das Destillat der Fraktionierung in einer Säule unterworfen wird, um am Kopf ein Destillat mit dem vorbestimmten Molekulargewicht zu erhalten, während die Fraktion mit einem größeren Molekulargewicht in den Crack-Reaktor zurückgeführt wird.

**4.** Verfahren nach Anspruch 3, worin der Crack-Katalysator aus Fluoriden oder Oxifluoriden der in Anspruch 1 angegebenen Metalle ausgewählt ist.